# EUROPÄISCHE PATENTSCHRIFT

(11) **EP 1 689 796 B1**
(45) Veröffentlichungstag und Bekanntmachung des Hinweises auf die Patenterteilung: **20.01.2010**
(21) Anmeldenummer: 04797969.5
(22) Anmeldetag: 18.11.2004
(51) Int. Cl.: C08F 220/00

(54) **(METH)ACRYLSÄUREESTER UNGESÄTTIGTER AMINOALKOHOLE UND DEREN HERSTELLUNG**
(METH)ACRYLIC ACID ESTERS OF UNSATURATED AMINOALCOHOLS AND PRODUCTION THEREOF
ESTERS D'ACIDE (METH)ACRYLIQUE D'AMINO-ALCOOLS NON SATURES ET LEUR PRODUCTION

(30) Priorität: 25.11.2003 DE 10355401
(43) Veröffentlichungstag der Anmeldung: 16.08.2006
(73) Patentinhaber: BASF SE, 67056 Ludwigshafen (DE)
(72) Erfinder: HERMELING, Dieter, 67459 Böhl-Iggelheim (DE); DANIEL, Thomas, 67165 Waldsee (DE); ELLIOTT, Mark, 67063 Ludwigshafen (DE); RIEGEL, Ulrich, 66849 Landstuhl (DE); DIETSCHE, Frank, 69198 Schriesheim (DE); SCHWALM, Reinhold, 67157 Wachenheim (DE)
(86) Internationale Anmeldenummer: PCT/EP2004/013064
(87) Internationale Veröffentlichungsnummer: WO 2005/058987

(56) Entgegenhaltungen:
- DE-A1- 3 716 543
- DE-A1- 3 818 426
- DE-A1- 10 358 372

## Beschreibung

Die vorliegende Erfindung betrifft (Meth)acrylsäureester ungesättigter Aminoalkohole, ein Verfahren zu deren Herstellung sowie deren Verwendung zur Herstellung von vernetzten quellbaren hydrogelbildenden Polymeren und vernetzte quellbare hydrogelbildende Polymere.

Der Begriff (Meth)acrylsäure bzw. (Meth)acrylsäureester steht in dieser Anmeldung für Methacrylsäure und Acrylsäure bzw. Methacrylsäureester und Acrylsäureester.

Quellbare hydrogelbildende Polymere, sogenannte Superabsorber (Super-Absorbing Polymers, SAP), sind aus dem Stand der Technik bekannt. Hierbei handelt es sich um Netzwerke flexibler hydrophiler Polymere, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber werden außerdem in anderen Gebieten der Technik eingesetzt, bei denen Flüssigkeiten, insbesondere Wasser oder wässrige Lösungen absorbiert werden, beispielsweise zu Lagerung, Verpackung, Transport, in Medizin und im Kosmetikbereich.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im, landwirtschaftlichen Gartenbau verwendet.

Gute Transporteigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Innenvernetzung erreicht, wodurch allerdings die Retention des Produktes verringert wird. Eine elegante Methode zur weiteren Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Die Patentanmeldung DE-A-196 46 484 beschreibt flüssigkeitsabsorbierende Polymere, die unter Verwendung einer Vernetzer/Monomerkombination aus drei Komponenten herstellbar sind, wobei es sich bei der ersten Komponente um eine Verbindung mit einer (Meth)allyl- und einer (Meth)acrylsäureesterfunktion, bei der zweiten Komponente um Mono(meth)acrylsäureester oder Mono(meth)allylalkoholether von Polyalkylenglykolen und bei der dritten Komponente um Ester ungesättigter Säuren mit Polyolen oder Di- bzw. Triallylamin oder Bisacrylamide handelt.

Die Patenanmeldung WO 90/15830 offenbart ein wasserquellbares Hydrokolloidpolymer, das ein einpolymerisiertes Vernetzergemisch enthält, das einen Bis- oder Trisacryloyl-haltigen ersten Vernetzer und einen unter Bisallylethern, -amiden, -aminen und Triallylamin ausgewählten zweiten Vernetzer umfasst.

In der Patentanmeldung WO 02/32964 wird ein Vernetzergemisch aus einem ersten Vernetzer mit wenigstens zwei (Meth)acrylestereinheiten pro Molekül und einem zweiten Vernetzer mit wenigstens zwei (Meth)allyloxyeinheiten pro Molekül zur Herstellung vernetzter, wasserquellbarer Polymere empfohlen.

Wasserquellbare Polymerisate können in wirtschaftlicher Weise in einem kontinuierlichen Verfahren hergestellt werden. So lehrt die Patentanmeldung EP-A-0 223 063 ein Verfahren zur kontinuierlichen Herstellung von vernetzten feinteiligen gelförmigen Polymerisaten in einem einwelligen zylindrischen Mischer, dessen Mischsegmente eine Förderung der Stoffe vom Anfang zum Ende des zylindrischen Mischers bewirken.

Die Patentanmeldung DE-A-199 55 861 beschreibt ein Verfahren zur kontinuierlichen Herstellung von vernetzten feinteiligen gelförmigen Polymerisaten, bei dem eine wässrige Lösung wasserlöslicher monoethylenisch ungesättigter Monomere und Vernetzer einem speziell ausgestaltetem Mischkneter zugeführt wird.

Der vorliegenden Erfindung liegt die Aufgabe zugrunde, vernetzte, wasserquellbare Polymere mit einem ausgewogenen Eigenschaftsprofil hinsichtlich Absorptionsvermögen, Gelfestigkeit, Aufnahmegeschwindigkeit und extrahierbaren Anteilen anzugeben, die sich außerdem vorteilhaft in einem kontinuierlichen Verfahren herstellen lassen.

Mit Triallylamin als Innenvernetzer werden wasserquellbare Polymere mit wenig extrahierbaren Anteilen erhalten, insbesondere bei der Polymerisation im neutralen oder basischem Medium. Nachteilig bei der Verwendung von Triallylamin sind aber die toxischen Eigenschaften von Triallylamin und der in Triallylamin enthaltenden Verunreinigungen (Monoallylamin und Diallylamin). Außerdem werden Allylamine schnell über die Haut resorbiert und sind flüchtig (Monoallylamin Sdp. 58°C; Diallylamin Sdp. 111°C; Triallylamin Sdp. 155°C).

Es bestand daher ein Bedarf nach Innenvernetzern, mit denen wasserquellbare Polymere mit geringem extrahierbaren Anteil hergestellt werden können, und die weniger toxisch und weniger flüchtig als Triallylamin sind.

Unter Vernetzung wird in dieser Schrift wenn nicht anders erwähnt die Gelvernetzung, Innenvernetzung oder Quervernetzung von linearem oder schwach vernetzten Polymer verstanden. Diese Vernetzung kann über radikalische oder kationische Polymerisationsmechanismen oder andere, beispielsweise Michael-Addition, Ver- oder Umesterungsmechanismen erfolgen, bevorzugt durch radikalische Polymerisation.

Vernetzte quellbare hydrogelbildende Polymere sind bevorzugt solche mit einer Absorption von 0,9 Gew.-%iger Kochsalzlösung von mindestens dem 10-fachen Eigengewicht bezogen auf eingesetztes Polymer, bevorzugt dem 20-fachen Eigengewicht. Diese Absorption wird bevorzugt auch unter einem Druck von beispielsweise 0,7 psi erreicht.

Es wurde nun gefunden, dass diese Aufgaben unter Verwendung neuer Vernetzer gelöst werden.

Demgemäß betrifft die Erfindung (Meth)acrylsäureester ungesättigter Aminoalkhole der allgemeinen Formel 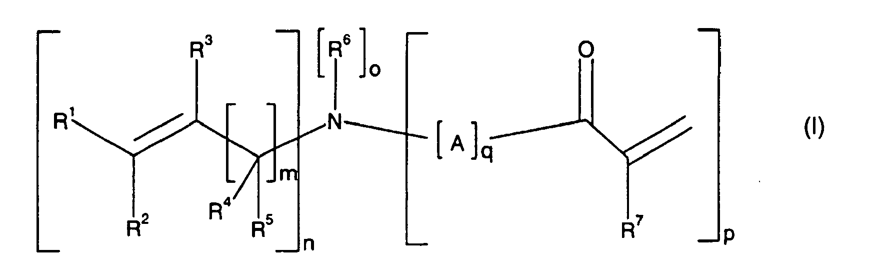 in der
- R¹, R², R³, R⁴ und R⁵: unabhängig voneinander Wasserstoff oder C₁- bis C₆-Alkyl, wobei C₃- bis C₆-Alkyl verzweigt oder unverzweigt sein können,
- R⁶: C₁- bis C₆-Alkyl, wobei C₃- bis C₆-Alkyl verzweigt oder unverzweigt sein können,
- R⁷: Wasserstoff oder Methyl,
- m: eine ganze Zahl von 0 bis 10,
- n: 1 oder 2,
- o: 0 oder 1,
- p: 1 oder 2 und
- q: eine ganze Zahl von 2 bis 100
bedeuten,
die Summe von n, o und p gleich 3 ist und

A für gleiche oder verschiedene Reste, ausgewählt aus der Gruppe 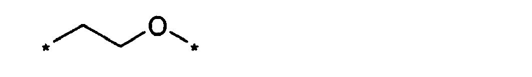 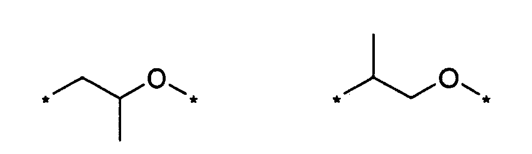 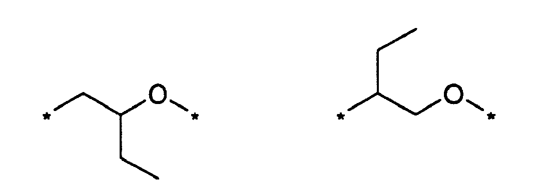 steht und mit *die Verknüpfungspositionen gekennzeichnet sind.

Bevorzugt sind (Meth)acrylsäureester ungesättigter Aminoalkhole der allgemeinen Formel 1, in der
- R¹, R², R³, R⁴ und R⁵: Wasserstoff,
- R⁶: C₁- bis C₃-Alkyl, wobei C₃-Alkyl verzweigt oder unverzweigt sein kann,
- R⁷: Wasserstoff oder Methyl,
- m: 0 oder 1,
- n: 1 oder 2,
- o: 0 oder 1,
- p: 1 oder 2 und
- q: eine ganze Zahl von 3 bis 40
bedeuten,
die Summe von n, o und p gleich 3 ist und

A für gleiche oder verschiedene Reste, ausgewählt aus der Gruppe 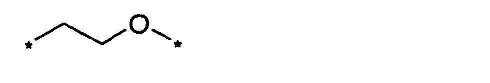 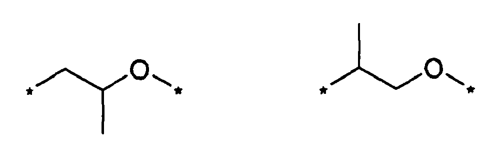 steht und mit * die Verknüpfungspositionen gekennzeichnet sind.

Besonders bevorzugt sind (Meth)acrylsäureester ungesättigter Aminoalkohole der allgemeinen Formel I, in der
- R¹, R², R³, R⁴ und R⁵: Wasserstoff,
- R⁷: Wasserstoff oder Methyl,
- m: 1,
- n: 1 oder 2,
- o: 0,
- p: 1 oder 2 und
- q: eine ganze Zahl von 5 bis 20
bedeuten,
die Summe von n, o und p gleich 3 ist und
A 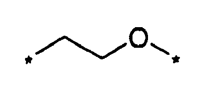 ist und mit * die Verknüpfungspositionen gekennzeichnet sind.

Die erfindungsgemäßen (Meth)acrylsäureester ungesättigter Aminoalkohole lassen sich beispielsweise aus einem Alkohol der allgemeinen Formel 11 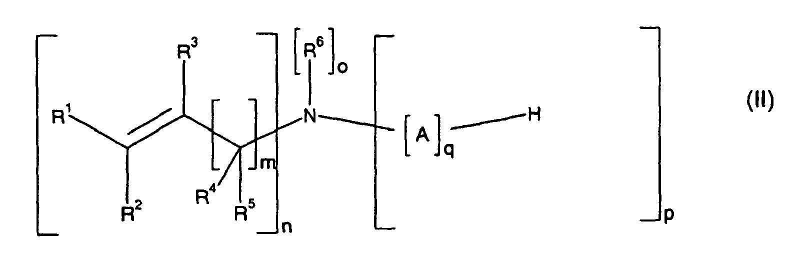 in der
R¹, R², R³, R⁴, R⁵, R⁶, m, n, o, p, q und A die obengenannten Bedeutungen haben,
durch Umesterung mit (Meth)acrylsäureestern sowie durch Acidolyse mit (Meth)acrylsäurechlorid oder (Meth)acrylsäureanhydrid herstellen. Die Umesterung mit niederen (Meth)acrylsäureestern ist bevorzugt. Niedere (Meth)acrylsäureester sind (Meth)acrylsäureester mit einem niedrigeren Siedepunkt als der Zielester.

Derartige Umesterungen können beispielsweise enzymatisch, wie beispielsweise in der Patentanmeldung EP-A-0 999 229 beschrieben, oder basisch katalysiert, wie beispielsweise in der Patentschrift GB-1,112,439 beschrieben, hergestellt werden.

Als ungesättigte Aminoalkohole einsetzbar sind alkoxilierte ungesättigte Amine, die durch Umsetzung eines ungesättigten Amins mit mindestens einem Alkylenoxid erhältlich sind.

Geeignete ungesättigte Amine sind beispielsweise Diallylamin, Allylamin, Methylallylamin, Ethylallylamin, Propylallylamin, Divinylamin, Vinylamin, Methylvinylamin, Ethylvinylamin und Propylvinylamin. Bevorzugt sind Diallylamin, Allylamin und Methylallylamin und ganz besonders bevorzugt ist Diallylamin.

Geeignete Alkylenoxide sind beispielsweise Ethylenoxid, Propylenoxid und/oder Butylenoxid.

Die Alkylenoxidkette kann bevorzugt aus Ethylenoxid-, Propylenoxid- und/oder Butylenoxideinheiten zusammengesetzt sein. Eine solche Kette kann sich aus einer Spezies eines Alkylenoxides oder aus einem Gemisch von Alkylenoxiden zusammensetzen. Wird ein Gemisch verwendet, können die unterschiedlichen Alkylenoxideinheiten statistisch oder als Block oder Blöcke einzelner Spezies vorliegen. Bevorzugt ist als Alkylenoxid Ethylenoxid, Propylenoxid oder ein Gemisch daraus, besonders bevorzugt ist Ethylenoxid oder ein Gemisch aus Ethylenoxid und Propylenoxid und ganz besonders bevorzugt Ethylenoxid.

Die bevorzugte Anzahl der Alkylenoxideinheiten in jeder Kette ist abhängig von der Anzahl der Ketten und beträgt
- für p = 2 von 2 bis 100, bevorzugt von 3 bis 30, besonders bevorzugt von 5 bis 15
   und
- für p = 1 von 2 bis 100, bevorzugt von 5 bis 40, besonders bevorzugt von 8 bis 20
   Alkylenoxideinheiten in jeder Kette.

Die angegebenen Alkoxylierungsgrade beziehen sich dabei jeweils auf den mittleren Alkoxylierungsgrad.

Selbstverständlich liegen herstellungsbedingt in der Regel Gemische vor, in denen zusätzlich niedere und höhere Oligomere enthalten sein können.

Die Umsetzung der ungesättigten Amine mit einem Alkylenoxid ist dem Fachmann an sich bekannt. Mögliche Durchführungsformen finden sich in Houben-Weyl, Methoden der Organischen Chemie, 4. Auflage, 1963, Thieme Verlag Stuttgart, Band 14/2, Seiten 440 bis 444.

Werden gemischt alkoxilierte ungesättigte Amine verwendet, so können die darin enthaltenen unterschiedlichen Alkoxygruppen zueinander im molaren Verhältnis von beispielsweise 0,05 - 20 : 1, bevorzugt 0,1 -10 : 1, und besonders bevorzugt 0,2 - 5 : 1, stehen.

An die Viskosität der erfindungsgemäß einsetzbaren ungesättigten Amine werden keine besonderen Ansprüche gestellt, außer daß sie bei einer Temperatur bis zu ca. 80°C problemlos pumpbar sein sollten, bevorzugt sollten sie eine Viskosität unter 1000 mPas aufweisen, bevorzugt unter 800 mPas, und ganz besonders bevorzugt unter 500 mPas.

Erfindungsgemäß einsetzbare (Meth)acrylsäureester sind beispielsweise Methylacrylat, Methylmethacrylat, Ethylacrylat, Ethylmethacrylat, n-Propylacrylat, n-Propylmethacrylat, Isopropylacrylat, Isopropylmethacrylat, n-Butylacrylat, n-Butylmethacrylat, Isobutylacrylat, lsobutylmethacrylat, 2-Ethylhexylacrylat und 2-Ethylhexylmethacrylat, vorzugsweise Methylacrylat, Ethylacrylat und n-Butylacrylat.

Als Umesterungskatalysatoren sind vor allem Titanalkoholate geeignet, deren Alkylgruppen C₁-C₄-Alkylreste darstellen, z.B. Tetramethyl-, Tetraethyl-, Tetraisopropyl-, Tetra-n-Propyl, Tetraisobutyl- und Tetra-n-Butyltitanat (siehe beispielsweise EP-B-0 298 867, EP-A-0 960 877). Weiterhin sind als Katalysatoren u.a. Titanphenolate (DE-A-20 08 618), Metallchelatverbindungen von z.B. Hafnium, Titan, Zirkon oder Calcium, Alkali- und Magnesiumalkoholate, organische Zinnverbindungen oder Calcium- und Lithiumverbindungen, beispielsweise Oxide, Hydroxyde, Carbonate oder Halogenide, geeignet. Auch stark basische Ionentauscher oder Zeolithe sind als Umesterungskatalysatoren einsetzbar. Bevorzugt sind Titanalkoholate und Natriumalkoholate. Ganz besonders bevorzugt sind Titanalkoholate und Natriumalkoholate, deren Alkoholkomponente der Alkoholkomponente des verwendeten niederen (Meth)acrylsäureesters entpricht.

Da es sich bei der Umesterung bekanntlich um eine Gleichgewichtsreaktion handelt, wird einer der Ausgangsstoffe im großen Überschuß eingesetzt und/oder eines der Reaktionsprodukte aus dem Gleichgewicht entfernt, um wirtschaftliche Umsätze zu erzielen. In der Regel wird daher der bei der Umesterung freigesetzte niedere Alkohol destillativ aus dem Gleichgewicht entfernt. Nachteilig ist dabei, daß die freigesetzten Alkohole, üblicherweise Methanol, Ethanol bzw. n-Butanol, mit den entsprechenden (Meth)acrylsäureestern (Methyl-, Ethyl- bzw. n-Butylacrylat) ein Azeotrop bilden und somit nicht direkt destillativ trennbar sind.

Aus ökologischen und ökonomischen Gründen ist die Rückführung des abdestillierten Gemischs oder Azeotrops bzw. seiner einzelnen Komponenten (Alkohol und/oder (Meth)acrylsäureester) in die Herstellung des (Meth)acrylsäureesters (beispielsweise Methyl-, Ethyl- bzw. n-Butylacrylat) vorteilhaft.

Als Polymerisationsinhibitoren einsetzbar sind beispielsweise Phenole wie Alkylphenole, wie beispielsweise o-, m- oder p-Kresol (Methylphenol), 2-tert.-Butyl-4-methylphenol, 2,6-Di-tert.-Butyl-4-methylphenol, 2-tert.-Butylphenol, 4-tert.-Butylphenol, 2,4-Di-tert.-butylphenol, Hydrochinon, Brenzkatechin (1,2-Dihydroxybenzol), Aminophenole, wie beispielsweise para-Aminophenol, Nitrosophenole, wie beispielsweise para-Nitrosophenol, p-Nitroso-o-kresol, Alkoxyphenole, wie beispielsweise 2-Methoxyphenol (Guajacol, Brenzkatechinmonomethylether), 2-Ethoxyphenol, 2-lsopropoxyphenol, 4-Methoxyphenol (Hydrochinonmonomethylether), Tocopherole, wie beispielsweise α-Tocopherol, β-Tocopherol, γ-Tocopherol, δ-Tocopherol und ε-Tocopherol , Tocol, α-Tocopherolhydrochinon, Chinone und Hydrochinone, wie beispielsweise Hydrochinon oder Hydrochinonmonomethylether, 2,5-Di-tert.-Butylhydrochinon, Benzochinon, Diphenylethen, N-Oxyle, wie beispielsweise 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, aromatische Amine, wie beispielsweise Phenylendiamine, N,N-Diphenylamin, N-Nitroso-diphenylamin, Nitrosodiethylanilin, N,N'-Dialkyl-para-phenylendiämin, wobei die Alkylreste gleich oder verschieden sein können und jeweils unabhängig voneinander aus 1 bis 4 Kohlenstoffatomen bestehen und geradkettig oder verzweigt sein können, wie beispielsweise N,N'-Di-iso-butyl-p-phenylendiamin, N,N'-Di-iso-propyl-p-phenylendiamin, Hydroxylamine, wie beispielsweise N,N-Diethylhydroxylamin, Harnstoffderivate, wie beispielsweise Harnstoff oder Thioharnstoff, phosphorhaltige Verbindungen, wie beispielsweise Triphenylphosphin, Triphenylphosphit, hypophosphorige Säure oder Triethylphosphit, schwefelhaltige Verbindungen, wie beispielsweise Diphenylsulfid, Phenothiazin oder Metallsalze, wie beispielsweise Kupfer-, Mangan-, Cer-, Nickel-, Chrom-chlorid, -dithiocarbamat, -sulfat, -salicylat oder -acetat oder Gemische davon.

Bevorzugt sind die genannten Phenole und Chinone, besonders bevorzugt sind Hydrochinon, Hydrochinonmonomethylether, 2-tert.-Butyl-4-methylphenol, 2,6-Di-tert.-butyl-4-methylphenol, 2,4-Di-tert.-butylphenol, α-Tocopherol, Diphenylethen, Triphenylphosphit, hypophosphorige Säure, CuCl₂ und Guajacol.

Besonders bevorzugt sind Hydrochinonmonomethylether, α-Tocopherol, Hydrochinon, und Alkylphenole, Diphenylethen, gegebenenfalls in Kombination mit Triphenylphosphit und/oder hypophosphoriger Säure.

Ganz besonders bevorzugt sind Hydrochinonmonomethylether, α-Tocopherol, Diphenylethen oder Gemische davon.

Zur weiteren Unterstützung der Stabilisierung kann ein sauerstoffhaltiges Gas, bevorzugt Luft oder ein Gemisch aus Luft und Stickstoff (Magerluft) anwesend sein. Besonders bevorzugt enthält das sauerstoffhaltige Gas weniger als 10 Vol.% Sauerstoff, ganz besonders bevorzugt 4 bis 6 Vol.% Sauerstoff.

Unter den aufgeführten Stabilisatoren sind solche bevorzugt, die aerob sind, d.h. solche, die zur Entfaltung ihrer vollen Inhibitorwirkung die Anwesenheit von Sauerstoff erfordern.

Selbstverständlich können bei der Umesterung auch Lösungsmittel eingesetzt werden besonders solche, die sich zur azeotropen Entfernung des Alkohols eignen, vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Ganz besonders bevorzugt wird aber auf ein Lösungsmittel bei der Umesterung verzichtet und der zur Umesterung verwendete niedere (Meth)acrylsäureester im Überschuß eingesetzt.

Im allgemeinen kann die Umesterung wie folgt durchgeführt werden:

Die Umesterungsapparatur besteht beisielsweise aus einem gerührten Reaktor, bevorzugt aus einem Reaktor mit Umlaufverdampfer und einer aufgesetzten Destillationseinheit.

Bei dem Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf, d.h. unter Verwendung einer Pumpe, besonders bevorzugt Naturumlauf, bei dem der Kreislaufstrom ohne mechanische Hilfsmittel bewerkstelligt wird, eingesetzt.

Selbstverständlich kann die Reaktion auch in mehreren Reaktionszonen, beispielsweise einer Reaktorkaskade aus zwei bis vier, bevorzugt zwei bis drei Reaktoren durchgeführt werden.

Geeignete Umlaufverdampfer sind dem Fachmann bekannt und beispielsweise beschrieben in R. Billet, "Verdampfertechnik", HTB-Verlag, Bibliographisches Institut Mannheim, 1965, Seite 53. Beispiele für Umlaufverdampfer sind Rohrbündelwärmetauscher, Plattenwärmetauscher, etc.

Selbstverständlich können im Umlauf auch mehrere Wärmetauscher vorhanden sein.

Die Destillationseinheit ist von an sich bekannter Bauart. Dabei kann es sich um eine einfache Destillation handeln, die gegebenenfalls mit einem Spritzschutz ausgestattet ist, oder um eine Rektifikationskolonne. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator ist von herkömmlicher Bauart.

Der niedere (Meth)acrylsäureester und der ungesättigte Aminoalkohol werden in der Umesterung in der Regel im molaren Überschuß wie oben angegeben bezogen auf die Hydroxygruppen des Alkohols eingesetzt. Der eingesetzte Überschuß kann bis zu ca. 1000:1 beitragen, falls gewünscht.

Als Umesterungskatalysatoren kommen die oben angeführten in Frage.

Sie werden in der Regel in einer Menge von 0,1 bis 5 Gew.-%, bezogen auf das Umesterungsgemisch, eingesetzt, bevorzugt 0,5 bis 5 Gew.-%, besonders bevorzugt 1 bis 4 Gew.-%, und ganz besonders bevorzugt 2 bis 4 Gew.-%.

Falls erforderlich kann der Umesterungskatalysator aus dem Reaktionsgemisch mit Hilfe eines lonenaustauschers entfernt werden. Der lonenaustauscher kann dabei direkt in das Reaktionsgemisch gegeben und anschließend abfiltriert oder das Reaktionsgemisch kann über eine lonenaustauscherschüttung geleitet werden.

Handelt es sich jedoch bei dem Katalysator um einen suspendierten lonentauscher, so wird dieser bevorzugt durch Filtration entfernt.

Das Polymerisationsinhibitor(gemisch) wird im allgemeinen in einer Gesamtmenge von 0,01 bis 1 Gew.-%, bezogen auf das Umesterungsgemisch, eingesetzt, bevorzugt 0,02 bis 0,8 Gew.-%, besonders bevorzugt 0,05 bis 0,5 Gew.-%.

Das Polymerisationsinhibitor(gemisch) kann beispielsweise als alkoholische Lösung oder als Lösung in einem Edukt oder Produkt eingesetzt werden.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert und/oder durch das Reaktionsgemisch und/oder über dieses geleitet.

Der bei der Reaktion entstehende niedere Alkohol kann während oder nach der Umesterung abdestilliert werden, wobei dieser Vorgang durch ein mit dem Alkohol ein Azeotrop bildendes Lösungsmittel unterstützt werden kann.

Als Lösungsmittel zur azeotropen Entfernung des Alkohols, falls gewünscht, eignen sich die oben angeführten Kohlenwasserstoffe oder der im Überschuß eingesetzte niedere (Meth)acrylsäureester.

Bevorzugt ist die Durchführung der Umesterung in Gegenwart eines Überschusses an niederem (Meth)acrylsäureester.

Falls der aus dem Reaktionsgemisch zu entfernende niedere Alkohol nicht über ein azeotropbildendes Lösungsmittel entfernt wird, so ist es möglich, dieses über Strippen mit einem inerten Gas, bevorzugt einem sauerstoffhaltigen Gas, besonders bevorzugt mit Luft oder Magerluft zu entfernen.

Die Reaktionstemperatur der Umesterung liegt allgemein bei 40 bis 160°C, bevorzugt bei 60 bis 140°C, und besonders bevorzugt bei 80 bis 120°C. Die Temperatur kann im Reaktionsverlauf gleichbleiben oder ansteigen, bevorzugt wird sie im Reaktionsverlauf angehoben. In diesem Fall ist die Endtemperatur der Umesterung um 5 bis 30°C höher als die Anfangstemperatur. Die Temperatur der Umesterung kann durch Variation der Lösungsmittelkonzentration im Reaktionsgemisch oder durch Variation des Reaktionsdruckes geregelt werden.

Das Destillat kann, nach Kondensation, wahlweise entfernt werden und/oder als Rücklauf in die Destillationseinheit geführt werden und/oder direkt in die Reaktionszone geleitet werden und/oder in einen Umlaufverdampfer geführt werden, wie in der Patentanmeldung WO 02/50014 beschrieben.

Der Rücklauf kann, wie in der Patentanmeldung DE-A 199 41 136 beschrieben, zur Steuerung der Temperatur in der Umesterung verwendet werden.

Die Umesterung kann drucklos aber auch bei Überdruck oder Unterdruck durchgeführt werden, vorzugsweise wird bei Normaldruck oder Unterdruck, besonders bevorzugt bei einem Reaktionsdruck von 200 bis 1013 mbar, gearbeitet.

Die Reaktionszeit beträgt in der Regel 2 bis 20 Stunden, vorzugsweise 4 bis 15 und besonders bevorzugt 7 bis 12 Stunden.

Die Reihenfolge der Zugabe der einzelnen Reaktionskomponenten ist nicht wesentlich. Es können alle Komponenten gemischt vorgelegt und anschließend aufgeheizt werden oder es können eine oder mehrere Komponenten nicht oder nur teilweise vorgelegt und erst nach dem Aufheizen zugegeben werden.

Wird die Umesterung in einem Reaktor mit Naturumlaufverdampfer durchgeführt, so ist es zweckmäßig die niedriger siedenden Reaktionskomponenten zumindest teilweise vorzulegen.

Der einsetzbare niedere (Meth)acryläureester ist in seiner Zusammensetzung nicht beschränkt und kann im Fall von n-Butylacrylat beispielsweise folgende Komponenten aufweisen:

| | |
|---|---|
| n-Butylacrylat | 98 - 99,9 Gew% |
| n-Buylacetat | 0,01 -0,1 Gew% |
| n-Butylpropionat | 0,01 - 0,1 Gew% |
| Wasser | 0,001 - 0,05 Gew% |
| Carbonylhaltige | 0,001 - 0,03 Gew% |
| Acrylsäure | 0,001 - 0,01 Gew% |
| Inhibitoren | 0,001 - 0,002 Gew% |

Der eingesetzte (Meth)acrylsäureester ist in der Regel stabilisiert mit 10 bis 20 ppm Hydrochinonmonomethylether oder anderen Stabilisatoren in Mengen, die eine vergleichbare Stabilisierung ermöglichen. Unter dem Ausdruck Carbonylhaltige werden hier beispielsweise Aceton und niedere Aldehyde, wie z.B. Formaldehyd, Acetaldehyd, Crotonaldehyd, Acrolein, 2- und 3- Furfural und Benzaldehyd, verstanden.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) von unten in den Umlaufverdampfer geleitet werden, beispielsweise in Mengen von 0,1 bis 1 m³/m³h, bevorzugt 0,2 bis 0,8 m³/m³h, und besonders bevorzugt 0,3 bis 0,7 m³/m³h, bezogen auf das Volumen des Reaktionsgemisches.

Der Verlauf der Umesterung kann durch Verfolgung der ausgetragenen Alkoholmenge verfolgt werden.

Die Reaktion kann beispielsweise beendet werden, sobald 90 % der theoretisch zu erwartenden Alkoholmenge ausgetragen worden sind, bevorzugt bei mindestens 95% und besonders bevorzugt bei mindestens 98%.

Nach Beendigung der Umesterung wird der Umesterungskatalysator auf übliche Weise zerstört. Dies geschieht beispielsweise bei Titanalkoholaten durch Zusatz geringer Mengen an Wasser, dadurch entstehen abfiltrierbare Titandioxidausfällungen, oder bei Alkalialkoholaten durch Neutralisation, vorzugsweise mit (Meth)acrylsäure.

In einer weiteren Ausführungsform kann die Reaktionsmischung nach beendeter Umesterung mit Wasser verdünnt werden und auf eine Konzentration von beispielsweise 10 bis 90 Gew.-%, bevorzugt 20 bis 80 Gew.-%, besonders bevorzugt 20 bis 60 Gew.- %, ganz besonders bevorzugt 30 bis 50 Gew.-%, und insbesondere ca. 40 Gew.%, verdünnt werden, beispielsweise um die Viskosität zu verringern.

Falls erforderlich kann das Reaktionsgemisch einer Entfärbung, beispielsweise durch Behandlung mit Aktivkohle oder Metalloxiden, wie beispielsweise Aluminiumoxid, Siliciumoxid, Magnesiumoxid, Zirkonoxid, Boroxid oder Gemischen davon, in Mengen von beispielsweise 0,1 bis 50 Gew.-%, bevorzugt 0,5 bis 25 Gew.-%, besonders bevorzugt 1 bis 10 Gew.-%, bei Temperaturen von beispielsweise 10 bis 100°C, bevorzugt 20 bis 80°C, und besonders bevorzugt 30 bis 60°C, unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Neutralisation oder nach Entfernung des nicht umgesetzten niederen (Meth)acrylsäureesters.

Der im Überschuß für die Umesterung eingesetzte und im Reaktionsgemisch enthaltene niedere (Meth)acrylsäureester kann durch Destillation im wesentlichen entfernt werden. Weitere im Rohester enthaltende niedrig siedende Komponenten, beispielsweise Lösungsmittel, werden gleichzeitig entfernt.

Die destillative Abtrennung der Hauptmenge des niederen (Meth)acrylsäureesters erfolgt beispielsweise in einem Rührkessel mit Doppelwandheizung und/oder innenliegenden Heizschlangen unter vermindertem Druck, beispielsweise bei 20 bis 700 mbar, bevorzugt 30 bis 500 mbar, und besonders bevorzugt 50 bis 150 mbar, und einer Temperatur von 40 bis 120°C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 bis 700 mbar, bevorzugt 30 bis 500 mbar, und besonders bevorzugt 50 bis 150 mbar, und einer Temperatur von 40 bis 120°C durch den Apparat geführt.

Bei der Verwendung von Wasser als Verdünnungsmittel kann nicht umgesetzter (Meth)acrylsäureester azeotrop abdestilliert werden. Dabei kann das Destillat, nach Kondensation, in einen Phasentrennapparat geführt werden. Die so erhaltene organische Phase kann ausgeschleust werden, die wässrige Phase kann ebenfalls ausgeschleust oder als Rücklauf in die Destillationseinheit geführt werden.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 bis 1 m³/m³h, bevorzugt 0,2 bis 0,8 m³/m³h, und besonders bevorzugt 0,3 bis 0,7 m³/m³h, bezogen auf das Volumen des Reaktionsgemisches.

Der Gehalt an Ausgangsester im Rückstand beträgt nach der Destillation in der Regel unter 5 Gew.-%, bevorzugt 0,5 bis 5 Gew.%, und besonders bevorzugt 1 bis 3 Gew.- %.

Der abgetrennte (Meth)acylsäureester kann kondensiert und bevorzugt wiederverwendet werden.

Falls erforderlich kann zusätzlich oder anstelle der Destillation eine Lösungsmittelstrippung durchgeführt werden.

Dazu wird der Zielester, der noch geringe Mengen an niederem (Meth)acrylsäureester und/oder organisches Lösungsmittel enthält, auf 50 bis 90°C. bevorzugt 80 bis 90°C, erwärmt und die restlichen Leichtsieder mit einem geeigneten Gas in einer geeigneten Apparatur entfernt. Zur Unterstützung kann gegebenenfalls auch Vakuum angelegt werden.

Geeignete Apparaturen sind beispielsweise Kolonnen von an sich bekannter Bauart, die die üblichen Einbauten, beispielsweise Böden, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie beispielsweise ein Luwa-, Rotafilm- oder Sambayverdampfer, der als Spritzschutz beispielsweise mit einem Demister ausgerüstet sein kann.

Geeignete Gase sind unter den Strippbedingungen inerte Gase, bevorzugt sauerstoffhaltige Gase, besonders bevorzugt Luft oder Gemische aus Luft und Stickstoff (Magerluft) oder Wasserdampf, insbesondere solche, die auf 50 bis 100°C temperiert sind.

Die Strippgasmenge beträgt beispielsweise 5 bis 20 m³/m³h, besonders bevorzugt 10 bis 20 m³/m³h, und ganz besonders bevorzugt 10 bis 15 m³/m³h, bezogen auf das Volumen des Reaktionsgemisches.

Falls notwendig kann der (Meth)acrylsäureester des ungesättigten Aminoalkohols in einem beliebigen Stadium des Aufarbeitungsverfahrens, bevorzugt nach erfolgter Leichtsiederentfernung, einer Filtration unterworfen werden, um ausgefallene Spuren an Salzen sowie gegebenenfalls enthaltenes Entfärbungsmittel zu entfernen.

Die nach dem obigen Verfahren erhältlichen (Meth)acrylsäureester ungesättigter Aminoalkohole und erfindungsgemäßen wässrigen Lösungen können Verwendung finden
- als Radikalvernetzer von wasserabsorbierenden Hydrogelen,
- als Ausgangsstoff für die Herstellung von Polymerdispersionen,
- als Ausgangsstoff für die Herstellung von Polyacrylaten (außer Hydrogelen),
- als Lackrohstoff oder
- als Zementadditiv.

Zur Verwendung als Radikalvernetzer von wasserabsorbierenden Hydrogelen sind insbesondere solche erfindungsgemäßen (Meth)acrylsäureester ungesättigter Aminoalkohole geeignet, die eine Wasserlöslichkeit (bei 25°C in destilliertem Wasser) von mindestens 5 Gew.-%, bevorzugt mindestens 10 Gew.-%, besonders bevorzugt mindestens 20 Gew.-%, ganz besonders bevorzugt mindestens 30 Gew.-%, und insbesondere von mindestens 50 Gew.-% aufweisen.

Zur Herstellung der vernetzten quellbaren hydrogelbildenen Polymere geeignete hydrophile Monomere sind beispielsweise polymerisationsfähige Säuren, wie Acrylsäure, Methacrylsäure, Ethacrylsäure, α-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide. Die Monomeren können allein oder in Mischung untereinander eingesetzt werden. Des weiteren wasserlösliche N-Vinylamide oder auch Diallyldimethylammoniumchlorid.

Besonders bevorzugte hydrophile Monomere sind Acrylsäure und Methacrylsäure.

Zur Optimierung von Eigenschaften kann es sinnvoll sein, zusätzliche monoethylenisch ungesättigte Verbindungen einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäuren, wie beispielsweise Acrylamid, Methacrylamid und N-Vinylformamid, N-Vinylacetamid, N-Methyl-vinylacetamid, Acrylnitril und Methacrylnitril. Weitere geeignete Verbindungen sind beispielsweise Vinylester von gesättigten C₁- bis C₄-Carbonsäuren wie Vinylformiat, Vinylacetat oder Vinylpropionat, Alkylvinylether mit mindestens zwei C-Atomen in der Alkylgruppe, wie beispielsweise Ethylvinylether oder Butylvinylether, Ester von monoethylenisch ungesättigten C₃- bis C₆-Carbonsäuren, wie beispielsweise Ester aus einwertigen C₁- bis C₁₈-Alkoholen und Acrylsäure, Methacrylsäure oder Maleinsäure, Halbester von Maleinsäure, wie beispielsweise Maleinsäure-monomethylester, N-Vinyllactame wie N-Vinylpyrrolidon oder N-Vinylcaprolactam, Acrylsäure- und Methacrylsäureester von alkoxilierten einwertigen, gesättigten Alkoholen, wie beispielsweise von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen (Mₙ) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, wie beispielsweise Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-%, zugesetzt.

Bevorzugt bestehen die vernetzten (Co)Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 bis 40 Gew.-% bezogen auf ihr Gesamtgewicht keine säuregruppentragenden monoethylenisch ungesättigten Monomeren.

Die Herstellung (meth)acrylsäurehaltiger (Co)Polymere, Polyacrylsäuren und Superabsorbern ist vielfach beschrieben, siehe beispielsweise "Modern Superabsorbent Polymer Technology", F.L. Buchholz and A.T. Graham, Wiley-VCH, 1998, Seiten 69 bis 117.

Bevorzugt sind solche Hydrogele, die durch vernetzende Polymerisation oder Copolymerisation von säuregruppentragenden monoethylenisch ungesättigten Monomeren oder deren Salzen erhalten werden.

Bei dem Verfahren zur Nachvernetzung wird das Ausgangspolymer mit einem Nachvernetzer behandelt und bevorzugt während oder nach dem Behandeln durch Temperaturerhöhung nachvernetzt und getrocknet, wobei der Vernetzer bevorzugt in einem inerten Lösemittel enthalten ist. Unter inerten Lösemittel werden solche verstanden, die bei der Reaktion weder mit dem Ausgangspolymer noch mit dem Nachvernetzer im wesentlichen reagieren. Bevorzugt sind solche Lösemittel, die zu mehr als 90%, bevorzugt mehr als 95%, besonders bevorzugt mehr als 99%, insbesondere zu mehr als 99,5%, nicht chemisch mit dem Ausgangspolymer oder Nachvernetzer reagieren.

Bevorzugt zur Nachvernetzung und Trocknung ist dabei der Temperaturbereich zwischen 30 und 250°C, insbesondere 50 und 200°C, ganz besonders bevorzugt ist der Bereich zwischen 100 und 180°C. Die Aufbringung der Oberflächennachvernetzungslösung erfolgt bevorzugt durch Aufsprühen auf das Polymere in geeigneten Sprühmischern. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BE-PEX-Mischer, NAUTA-Mischer, SHUGGI-Mischer oder PROCESSALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie beispielsweise ein Hordentrockner, ein Drehrohrofen, oder eine beheizbare Schnecke. Es kann aber auch beispielsweise eine azeotrope Destillation als Trocknungsverfahren benutzt werden. Die bevorzugte Verweilzeit bei dieser Temperatur im Reaktionsmischer oder Trockner beträgt unter 60 Minuten, besonders bevorzugt unter 30 Minuten.

Bevorzugt sind obige Verfahren, wobei das Ausgangspolymere eine polymere (Meth)acrylsäure oder ein Poly(meth)acrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Bevorzugt werden solche Verfahren in denen der Radikalvernetzer in einer Dosierung von 0,01 bis 5,0 Gew.-%, bevorzugt 0,02 bis 3,0 Gew.%, ganz besonders bevorzugt 0,03 bis 2,5 Gew.%, insbesondere 0,05 bis 1,0 Gew.-%, und speziell 0,1 bis 0,75 Gew.-%, bezogen auf das Ausgangspolymer verwendet wird.

Gegenstand der Erfindung sind auch Polymere, hergestellt nach einem der oben genannten Verfahren und deren Verwendung in Hygieneartikeln, Verpackungsmaterialien und in Nonwovens, sowie die Verwendung von einem oben genannten Stoffgemisch zur Herstellung von vernetzten oder durch Wärmebehandlung vernetzungsfähigen Polymeren, insbesondere in Lacken und Farben.

Die dabei einzusetzenden vernetzten quellbaren hydrogelbildenen Polymere (Ausgangspolymere) sind insbesondere Polymere aus (co)polymerisierten hydrophilen Monomeren, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Diese Hydrogele sind dem Fachmann bekannt und beispielsweise beschrieben in US-4,28, 082, DE-C-27 06 135, US-4,340,706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A-26 12 846, DE-A-40 20 780 EP-A-0 205 674, US-5,145,906, EP-A-0 530 438, EP-A-0 670 073, US-4,057,521, US-4,062,817, US-4,525,527, US-4,295,987, US-5,011,892, US-4,076,663 oder US-4,931,497. Besonders geeignet sind auch vernetzte quellbare hydrogelbildene Polymere aus einem Herstellprozeß wie in der Patentanmldung WO 01/38402 beschrieben, sowie anorganisch-organische hybride vernetzte quellbare hydrogelbildene Polymere wie in der Patentanmeldung DE-A-198 54 575 beschrieben. Der Inhalt der vorstehend genannten Patentdokumente, insbesondere die nach den Verfahren hergestellten Hydrogele, sind ausdrücklich Bestandteil der vorliegenden Offenbarung.

Geeignete Pfropfgrundlagen für vernetzte quellbare hydrogelbildene Polymere, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das vernetzte quellbare hydrogelbildene Polymer kann über radikalische Pfropfcopolymerisation von Acrylsäure oder Acrylat auf eine wasserlösliche Polymermatrix erhalten werden. Geeignete wasserlösliche Polymermatrices sind beispielsweise, aber nicht ausschliesslich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikalvernetzer eingesetzt.

Das vernetzte quellbare hydrogelbildene Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozeß ein in Wasser lösliches Silikat oder lösliches Aluminat oder Gemische von beiden eingesetzt wurde.

Bevorzugte vernetzte quellbare hydrogelbildene Polymere sind insbesondere Polyacrylate, Polymethacrylate sowie die in US-4,931,497, US-5,011,892 und US-5,041,496 beschriebene Pfropfpolymere. Ganz besonders bevorzugte vernetzte quellbare hydrogelbildene Polymere sind die in WO 01/38402 beschriebenen Kneterpolymere und die in DE-A-198 545 75 beschriebenen hybriden organisch-anorganischen vernetzten quellbaren hydrogelbildenen Polymere auf Basis von Polyacrylaten.

Die erfindungsgemäß hergestellten, als Radikalvernetzer in vernetzten quellbaren hydrogelbildenen Polymeren verwendbaren Substanzen können allein oder in Kombination mit anderen Vernetzern, beispielsweise Innen- oder Oberflächenvernetzern verwendet werden, beispielsweise den folgenden:

Methylenbisacryl- bzw. -methacrylamid, Ester ungesättigter Mono- oder Polycarbonsäuren von Polyolen, wie Diacrylat oder Triacrylat, z. B. Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weitere geeignete Covernetzer sind Pentaerythrittri- und -tetraallylether, Polyethylenglykoldiallylether, Monoethylenglykol-diallylether, Glyceroldi- und Triallylether, Polyallylether auf Basis Sorbitol, sowie ethoxilierte Varianten davon. Weiterhin besonders bevorzugte Covernetzer sind die Polyethylenglykol-diacrylate, ethoxilierte Derivate von Trimethylolpropantriacrylat wie beispielsweise Sartomer^{®} SR 9035 der Firma Sartomer, sowie ethoxilierte Derivate von Glycerindiacrylat und Glycerintriacrylat. Selbstverständlich können auch Gemische der obigen Vernetzer eingesetzt werden.

Ganz besonders bevorzugt sind solche vernetzten quellbaren hydrogelbildenden Polymere, die mit einem erfindungsgemäß hergestellten (Meth)acrylsäureester eines ungesättigten Aminoalkohols als einzigen Radikalvernetzer hergestellt werden.

Das vernetzte quellbare hydrogelbildene Polymer ist bevorzugt eine polymere Acrylsäure oder ein Polyacrylat

Die vernetzten quellbaren hydrogelbildenen Polymere können durch an sich bekannte Polymerisationsverfahren hergestellt werden. Bevorzugt ist die Polymerisation in wässriger Lösung nach dem Verfahren der sogenannten Gelpolymerisation. Dabei werden wie oben angeführt verdünnte, bevorzugt wässrige, besonders bevorzugt 15 bis 50 gew.-%ige wässrige Lösungen eines oder mehrerer hydrophiler Monomerer und gegebenenfalls einer geeigneten Pfropfgrundlage in Gegenwart eines Radikalinitiators bevorzugt ohne mechanische Durchmischung unter Ausnutzung des Trommsdorff-Norrish-Effektes (Makromolekulare Chemie, Band 1, Jahrgang 1947, Seiten 169 ff), polymerisiert. Die Polymerisationsreaktion kann im Temperaturbereich vonn 0 bis 150°C, vorzugsweise von 10 bis 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodüsobutyronitril sowie anorganische Peroxiverbindungen wie (NH₄)₂S₂O₈, K₂S₂O₈ oder H₂O₂.

Sie können gegebenenfalls in Kombination mit Reduktionsmitteln wie beispielsweise Ascorbinsäure, Natriumhydrogensulfit, und Eisen(II)- sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie beispielsweise Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich von 50 bis 130°C, vorzugsweise von 70 bis 100°C, können die Qualitätseigenschaften der Polymere noch verbessert werden.

Die erhaltenen Gele werden zu 0 bis 100 mol-%, bevorzugt zu 25 bis 100 mol-%, und besonders bevorzugt zu 50 bis 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden, wie oben beschrieben. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels UV-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

Das getrocknete vernetzte quellbare hydrogelbildene Polymer kann hiernach gemahlen und gesiebt werden, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 bis 1000 µm, bevorzugt bei 45 bis 850 µm, besonders bevorzugt bei 100 bis 800 µm, und ganz besonders bevorzugt bei 100 bis 700 µm. In diesen Bereichen liegen bevorzugt 80 Gew.-% der Teilchen, insbesondere 90 Gew.-% der Teilchen. Die Größenverteilung kann mit etablierten Lasermethoden bestimmt werden.

Gegenstand der vorliegenden Erfindung sind weiterhin vernetzte quellbare hydrogelbildende Polymere, die mindestens ein hydrophiles Monomer in einpolymerisierter Form enthalten und vernetzt sind mit einem (Meth)acrylsäureeester eines ungesättigten Aminoalkohols der Formel (I).

Bevorzugte (Meth)acrylsäureeester ungesättigter Aminoalkohole sind solche der Formel (1), wie oben definiert, in denen q unabhängig voneinander
- für p = 2 eine Zahl von 1 bis 100, bevorzugt von 3 bis 30, besonders bevorzugt von 5 bis 15
   und
- für p = 1 eine Zahl von 1 bis 100, bevorzugt von 5 bis 40, besonders bevorzugt von 8 bis 20
   ist.

Die durch die Formel (11) beschriebenen ungesättigten Aminoalkohole, deren (Meth)acrylsäureester als Vernetzer in den vorstehend genannten vernetzten quellbaren hydrogelbildenden Polymeren verwendet werden, sind jeweils alkoxiliert, vorzugsweise ethoxiliert, propoxiliert oder gemischt ethoxiliert und propoxiliert und insbesondere ethoxiliert oder gemischt ethoxiliert und propoxiliert und ganz besonders bevorzugt ausschließlich ethoxiliert.

Besonders bevorzugte (Meth)acrylsäureeester ungesättigter Aminoalkohole sind solche der Formel (I), deren ungesättigte Aminoalkohole sich von Diallylamin, Allylamin und Allylmethylamin ableiten.

Der CRC-Wert [g/g] der erfindungsgemäßen vernetzten quellbaren hydrogelbildenden Polymere kann nach den in der Beschreibung angegebenen Methoden gemessen werden und ist mindestens 9, bevorzugt größer 15, insbesondere größer 20, besonders bevorzugt größer 25, insbesondere größer 30, insbesondere bevorzugt größer 35.

Der AUL-0,7psi-Wert [g/g] der erfindungsgemäßen vernetzten quellbaren hydrogelbildenden Polymere kann nach den in der Beschreibung angegebenen Methoden gemessen werden und ist nach der Nachvernetzung bevorzugt größer 5, insbesondere größer 10, besonders bevorzugt größer 15, insbesondere größer 20, insbesondere bevorzugt größer 25.

Die vorliegende Erfindung betrifft ferner die Verwendung der oben genannten vernetzten quellbaren hydrogelbildenden Polymere in Hygieneartikeln. Beispielsweise kann der Hygieneartikel wie folgt aufgebaut sein:
(A) eine obere flüssigkeitsdurchlässige Abdeckung
(B) eine untere flüssigkeitsundurchlässige Schicht
(C) einen zwischen (A) und (B) befindlichen Kern, enthaltend 10 bis 100 Gew.-% des erfindungsgemäßem vernetzten quellbaren hydrogelbildenden Polymers 0 bis 90 Gew.-% hydrophiles Fasermaterial
   bevorzugt 30 bis 100 Gew.-% des erfindungsgemäßem vernetzten quellbaren hydrogelbildenden Polymers, 0 bis 70 Gew.-% hydrophiles Fasermaterial besonders bevorzugt 50 bis 100 Gew.-% des erfindungsgemäßem vernetzten quellbaren hydrogelbildenden Polymers 0 bis 50 Gew.-% hydrophiles Fasermaterial
   insbesondere bevorzugt 70 bis 100 Gew.-% des erfindungsgemäßem vernetzten quellbaren hydrogelbildenden Polymers, 0 bis 30 Gew.-% hydrophiles Fasermaterial
   am meisten bevorzugt 90 bis 100 Gew.-% des erfindungsgemäßem vernetzten quellbaren hydrogelbildenden Polymers, 0 bis 10 Gew.-% hydrophiles Fasermaterial
(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Unter Hygieneartikel sind dabei beispielsweise Inkontinenzeinlagen und Inkontinenzhosen für Erwachsene oder Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355, EP-A-1 023 883.

Die flüssigkeitsundurchlässige Schicht (B) besteht in der Regel aus einer Folie aus Polyethylen oder Polypropylen.

Der Kern (C) enthält neben dem erfindungsgemäßen vernetzten quellbaren hydrogelbildenden Polymer hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 µm, bevorzugt 10 bis 100 µm. Darüberhinaus haben die Fasern eine Mindestlänge von 1 mm.

Der Aufbau und die Form von Windeln ist allgemein bekannt und beispielsweise in der WO 95/26209 Seite 66, Zeile 34 bis Seite 69, Zeile 11, DE-A-196 04 601, EP-A-0 316 518 und EP-A-0 202 127 beschrieben. Allgemein werden Windeln und andere Hygieneartikel auch in WO 00/65084, insbesondere auf Seiten 6 bis 15, WO 00/65348, insbesondere auf Seiten 4 bis 17, WO 00/35502, insbesondere Seiten 3 bis 9, DE-A 197 37 434 und WO 98/08439 beschrieben. Hygieneartikel für die Damenhygiene werden in folgenden Literaturstellen beschrieben. Die erfindungsgemäßen wässrige Flüssigkeiten absorbierende vernetzten quellbaren hydrogelbildenden Polymere können dort eingestzt werden. Literaturstellen Damenhygiene: WO 95/24173: Absorption Article for Controlling Odour, WO 91/11977: Body Fluid Odour Control, EP-A-0 389 023: Absorbent Sanitary Articles, WO 94/25077: Odour Control Material, WO 97/01317: Absorbent Hygienic Article, WO 99/18905, EP-A-0 834 297, US-5,762,644, US-5,895,381, WO 98/57609, WO 00/65083, WO 00/69485, WO 00/69484, WO 00/69481, US-6,123,693, EP-A-1 104 666, WO 01/24755, WO 01/00115, EP-A-0105 373, WO 01/41692, EP-A-1 074 233. Tampons werden in folgenden Schriften beschrieben: WO 98/48753, WO 98/41179, WO 97/09022, WO 98/46182, WO 98/46181, WO 01/43679, WO 01/43680, WO 00/61052, EP-A-1 108 408, WO 01/33962, DE-A-100 20 662, WO 01/01910, WO 01/01908, WO 01/01909, WO 01/01906, WO 01/01905, WO 01/24729. Inkontinenzartikel werden in folgenden Schriften beschrieben: Disposable Absorbent Article for Incontinent Individuals: EP-A-0 311 344 Beschreibung Seiten 3 bis 9, Disposable Absorbent Article: EP-A-0 850 623, Absorbent Article: WO 95/26207, Absorbent Article: EP-A-0 894 502, Dry Laid Fibrous Structure: EP-A-0 850 616, WO 98/22063, WO 97/49365, EP-A-0 903 134, EP-A-0 887 060, EP-A-0 887 059, EP-A-0 887 058, EP-A-0 887 057, EP-A-0 887 056, EP-A-0 931 530, WO 99/25284, WO 98/48753. Damenhygiene- und Inkontinenzartikel wird in folgenden Schriften beschrieben: Catamenial Device: WO 93/22998 Beschreibung Seiten 26 bis 33, Absorbent Members for Body Fluids: WO 95/26209 Beschreibung Seiten 36 bis 69, Disposable Absorbent Article: WO 98/20916 Beschreibung Seiten 13 bis 24, Improved Composite Absorbent Structures: EP-A-0 306 262 Beschreibung Seiten 3 bis 14, Body Waste Absorbent Article: WO 99/45973. Diese Referenzen werden hiermit ausdrücklich in die Offenbarung der Erfindung einbezogen.

Die erfindungsgemäßen vernetzten quellbaren hydrogelbildenden Polymere eignen sich hervorragend als Absorptionsmittel für Wasser und wässrige Flüssigkeiten, so daß sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Zusätzlich zu den oben beschriebenen vernetzten quellbaren hydrogelbildenden Polymeren liegen in der absorbierenden Zusammensetzung gemäß der vorliegenden Erfindung Kompositionen vor, welche die vernetzten quellbaren hydrogelbildenden Polymere enthalten oder an denen sie fixiert sind. Jede Komposition ist geeignet, die die vernetzte quellbare hydrogelbildende Polymere aufnehmen kann und die darüber hinaus in die Absorptionsschicht integriert werden kann. Eine Vielzahl derartiger Zusammensetzungen ist bereits bekannt. Eine Komposition zum Einbau der vernetzten quellbaren hydrogelbildenden Polymere kann beispielsweise eine Fasermatrix sein, die aus einem Cellulosefasergemisch (airlaid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines airlaid web ist beispielsweise geschildert in der Patentanmeldung WO 98/28478. Des weiteren können offenporige Schäume oder ähnliches zum Einbau vernetzter quellbarer hydrogelbildender Polymere dienen.

Alternativ kann eine derartige Komposition durch Fusion zweier Einzelschichten entstehen, wobei eine oder besser eine Vielzahl an Kammern gebildet werden, die die vernetzten quellbaren hydrogelbildenden Polymere enthalten. Ein derartiges Kammersystem ist detailliert geschildert in der Patentanmeldung EP-A-0 615 736 Seite 7, Zeilen 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die vernetzten quellbaren hydrogelbildenden Polymerpartikel zurückzuhalten. Obige Beispiele zur Komposition der absorbierenden Zusammensetzung schließen auch Laminate aus mindestens zwei Schichten mit ein, zwischen die die vernetzten quellbaren hydrogelbildenden Polymere eingebaut und fixiert werden.

Generell besteht die Möglichkeit, Hydrogelpartikel innerhalb des Absorbent Cores zur Verbesserung der sogenannten Dry- und Wet-Integrity zu fixieren. Unter Dry- und Wet-Integrity versteht man die Fähigkeit, vernetzte quellbare hydrogelbildende Polymere derart in die absorbierende Zusammensetzung einzubauen, dass sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in der Patentanmeldung WO 95/26209, Seite 37, Zeile 36 bis Seite 41, Zeile 14. Besagte Passage ist somit Bestandteil dieser Erfindung. Methoden zur Erhöhung der Wet Strength finden sich auch in der Patentanmeldung WO 00/36216.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die vernetzten quellbaren hydrogelbildenden Polymere mit einem Gewichtsanteil von 10 bis 100 Gew.-%, bevorzugt 30 bis 100 Gew.-%, besonders bevorzugt 50 bis 100 Gew.-%, insbesondere bevorzugt 70 bis 100 Gew.-%, und am meisten bevorzugt 90 bis 100 Gew.- %, basierend auf dem Gesamtgewicht der Komposition und der vernetzten quellbaren hydrogelbildenden Polymere eingebaut.

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt die Patentanmeldung WO 95/26209, Seite 28, Zeile 9 bis Seite 36, Zeile 8. Besagte Passage ist somit Bestandteil dieser Erfindung.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetrafluorethylen, Polyvinylidenchlorid, Polyacrylverbindungen wie ORLON^{®}, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX^{®}), Polypropylenfasern und Polyethylen-Polypropylen-Zweikomponentenfasern, Polyesterfasern, wie Polyethylenterephthalatfasern (DAC-RON^{®} oder KODEL^{®}), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt von 75 bis 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9.000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10.000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner als 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90º ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die vernetzten quellbaren hydrogelbildenden Polymere am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie beispielsweise Polyethylenterephthalat (DACRON^{®}), und hydrophiles Nylon (HYDROFL^{®}). Geeignete hydrophile Fasern können auch erhalten werden durch Hydrophilierung hydrophober Fasern, wie beispielsweise die Behandlung thermoplastischer Fasern, erhalten aus Polyolefinen (beispielsweise Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die vernetzten quellbaren hydrogelbildenden Polymerpartikel werden in das beschriebene Fasermaterial eingebettet. Dies kann auf vielfältige Weise geschehen, indem man beispielsweise mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene^{®}557 H, Hercules, Inc. Wilmington Delaware, USA), Polyacrylamid- Überzüge (beschrieben in US-3,556,932 oder als Handelsprodukt der Marke Parez^{®} 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann beispielsweise die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind C₂-C₈ Dialdehyde, C₂-C₈ Monoaldehyde mit saurer Funktionalität, und insbesondere C₂-C₉ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens zwei Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, US-3,224,926, US-3,440,135, US-3,932,209, US-4,035,147, US-4,822,453, US-4,888,093, US-4,898,642 und US-5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie beispielsweise Verschmelzen durch Wärmebehandlung, Zugabe von Schmelzklebern, Latexbindern usw. miteinander verbunden.

Beispiele für Verfahren, mit denen man eine absorbierende Zusammensetzung erhält, die beispielsweise aus einem Trägermaterial bestehen, an den ein- oder beidseitig vernetzte quellbare hydrogelbildende Polymere fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteten vernetzten quellbaren hydrogelbildenden Polymere (c) besteht, wobei das Mischungsverhältnis von (100 bis 0) synthetische Faser: (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b)

in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so daß eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunden bis 3 Minuten, bevorzugt 1 Sekunde bis 1 Minute.

Die absorbierende Zusammensetzung wird im allgemeinen beispielsweise mit einer für Flüssigkeit durchlässien Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in der WO 95/26209.

Gegenstand der vorliegenden Erfindung sind weiterhin Hygieneartikel, enthaltend vernetzte quellbare hydrogelbildende Polymere, die mindestens ein hydrophiles Monomer in einpolymerisierter Form enthalten und vernetzt sind mit einem (Meth)acrylsäureeester eines ungesättigten Aminoalkohols der Formel (I).

Das erfindungsgemäße Verfahren wird durch die nachfolgenden Beispiele näher erläutert.

### Beispiele:

### Messmethoden

### Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106 - 850 µm) in einem 60 x 85 mm großen Teebeutel eingewogen, der anschließend verschweißt wird. Der Teebeutel wird für 30 Minuten in einen Überschuss von 0,9 gew.-%iger Kochsalzlösung gegeben (mindestens 0,83 1 Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

### Extrahierbare Anteile

Die Bestimmung des nach 16 Stunden extrahierbaren Anteils (Extractables 16h) erfolgte analog wie in EP-A-0 811 636, Seite 13, Zeilen 1 bis 19 beschrieben.

Herstellung der Allylaminpolyether

### Beispiel 1: Diallylamin - 5 EO

97 g Diallylamin werden mit 0,5 g KOH, 45 % in Wasser, in einem Autoklaven vorgelegt, dieser mit Argon gespült, und dann zusammen bei 80°C und reduziertem Druck (ca. 20 mbar) entwässert. Dann werden bei 145 bis 155°C 220 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 120°C nachgerührt. Nach Spülen mit Inertgas und Abkühlen auf 60°C wird der Katalysator durch Zugabe von Natriumpyrophosphat und anschließende Filtration abgetrennt.

### Beispiel 2: Diallylamin - 13 EO

Es wird wie in Beispiel 1 verfahren jedoch werden zu 97 g Diallylamin zunächst 44 g Ethylenoxid zudosiert, dann auf Raumtemperatur abgekühlt und mit 0,5 g KOH, 45% in Wasser versetzt. Hierbei bildet sich in bekannter Weise zunächst das Dialkylamin - 1 EO welches im folgenden analog Beispiel 1 weiter ethoxyliert wird.

Der Autoklav wird mit Argon gespült, und das Reaktionsgemisch dann bei 80°C und reduziertem Druck (ca. 20 mbar) entwässert. Dann werden bei 145 bis 155°C noch 528 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 120°C nachgerührt. Nach Spülen mit Inertgas und Abkühlen auf 60°C wird der Katalysator durch Zugabe von Natriumpyrophosphat und anschließende Filtration abgetrennt.

### Beispiel 3: Diallylamin - 18 EO

Es wird genau wie in Beispiel 1 verfahren jedoch werden zu 97 g Diallylamin mit 0,5 g KOH jetzt 792 g Ethylenoxid zugegeben.

### Beispiel 4: Monoallylamin - 5 EO

57 g Monoallylamin werden mit 0,5 g KOH, 45 % in Wasser, in einem Autoklaven vorgelegt, dieser mit Argon gespült, und dann zusammen bei 80°C und reduziertem Druck (ca. 20 mbar) entwässert. Dann werden bei 145 bis 155°C 220 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 120°C nachgerührt. Nach Spülen mit Inertgas und Abkühlen auf 60°C wird der Katalysator durch Zugabe von Natriumpyrophosphat und anschließende Filtration abgetrennt.

### Beispiel 5: Monoallylamin - 13 EO

Es wird genau wie in Beispiel 4 verfahren jedoch werden zu 57 g Monoallylamin mit 0,5 g KOH jetzt 572 g Ethylenoxid zugegeben.

Herstellvorschrift der ethoxylierten Acrylat-Allylamine

### Beispiel 6: Diallylamin - 5 EO - Monoacrylat

80 g des 5-fach ethoxylierten Diallylamins aus Beispiel 1 werden mit 215 g Methylacrylat, 10 g Novozym (immobilisierte Lipase aus Candida antarctica) und 0,4 g Hydrochinonmonomethylether vorgelegt und bei 60°C durch Umesterung verestert. Dabei wird der entstehende Methanol kontinuierlich bei 500-560 mbar abdestilliert wobei auch ein Teil des eingesetzten Methylacrylats übergeht. Nach ca. 8 Stunden wird daher nochmals, nach Bedarf, Methylacrylat und weitere 10 g Novozym nachdosiert und weitere 8 Stunden verestert. Das Rohprodukt wird durch Filtration vom Novozym-Katalysator getrennt, und anschliessend wird das überschüssige Methylacrylat am Rotationsverdampfer aus dem Produkt abdestilliert.

### Beispiele 7 bis 10:

Es wird genau wie in Beispiel 6 verfahren, jedoch werden die in der Tabelle 1 angegeben Einsatzmengen verwendet.

**Tabelle 1:**

| Beispiel | Allylaminpolyether | Methylacrylat* | Novozym* | Hydrochinon Monomethylether | Vitamin E** |
|---|---|---|---|---|---|
| 7 | 84 g Diallylamin - 13 EO aus Beispiel 2 | 108 g | 10 g | 0,3 g | 0,2 g |
| 8 | 111 g Diallylamin -18 EO aus Beispiel 3 | 108 g | 10 g | 0,3 g | 0,3 g |
| 9 | 70 g Monoallylamin - 5 EO aus Beispiel 4 | 216 g | 10 g | -- | 0,5 g |
| 10 | 79 g Monoallylamin - 13 EO aus Beispiel 5 | 108 g | 10 g | 0,4 g | 0,2 g |

| | | | | | |
|---|---|---|---|---|---|
| *) Einsatzmenge zu Beginn des Ansatzes, nach 8 Stunden wurde nochmals nach Bedarf analog Beispiel 6 Methylacrylat und Novozym nachdosiert. **) Vitamin E wird zusammen mit Hydrochinonmonomethylether vorgelegt. | | | | | |

### Herstellbeispiel Superabsorber:

Vergleichsbeispiel 1: Trimethylolpropan-1 5 EO-Triacrylat

In einen Lödige-Pflugscharkneter Typ VT 5R-MK (5 I Volumen) werden 388 g entionisiertes Wasser, 173,5 g Acrylsäure, 2033,2 g einer 37,3 Gew.%igen Natriumacrylatlösung (100 mol% neutralisiert) sowie 4,50 g des Vernetzers Trimethylolpropan-15 EO-Triacrylat (Sartomer® SR 9035, Fa. Sartomer) vorgelegt und unter Durchperlen von Stickstoff 20 Minuten inertisiert. Dann wird durch Zusatz (verdünnte wässrige Lösungen) von 2,112 g Natriumpersulfat, 0,045 g Ascorbinsäure sowie 0,126 g Wasserstoffperoxid bei ca. 23 °C gestartet. Nach dem Start wird die Temperatur des Heizmantels der Reaktionstemperatur im Reaktor mittels Regelung nachgeführt. Das letztlich erhaltene krümelige Gel wird dann bei 160°C ca. 3 h im Umlufttrockenschrank getrocknet. Anschliessend wird gemahlen und auf 300-600 Mikrometer abgesiebt. Das erhaltene Hydrogel wird hernach oberflächennach-vernetzt. Die Eigenschaften des erhaltenen Hydrogels sind in der nachfolgenden Tabelle zusammengefasst.

### Vergleichsbeispiel 2: Tripropylenglykoldiacrylat

In einen Lödige-Pflugscharkneter Typ VT 5R-MK (5 **I** Volumen) werden 388 g entionisiertes Wasser, 173,5 g Acrylsäure, 2033,2 g einer 37,3 Gew.%igen Natriumacrylatlösung (100 mol% neutralisiert) sowie 2,10 g des Vernetzers Tripropylenglykoldiacrylat (Laromer® TPGDA, Fa. BASF AG) vorgelegt und unter Durchperlen von Stickstoff 20 Minuten inertisiert. Dann wird durch Zusatz (verdünnte wässrige Lösungen) von 2,112 g Natriumpersulfat, 0,045 g Ascorbinsäure sowie 0,126 g Wasserstoffperoxid bei ca. 23 °C gestartet. Nach dem Start wird die Temperatur des Heizmantels der Reaktionstemperatur im Reaktor mittels Regelung nachgeführt. Das letztlich erhaltene krümelige Gel wird dann bei 160 °C ca. 3 h im Umlufttrockenschrank getrocknet. Anschliessend wird gemahlen und auf 300-600 Mikrometer abgesiebt. Das erhaltene Hydrogel wird hernach oberflächennach-vernetzt. Die Eigenschaften des erhaltenen Hydrogels sind in der nachfolgenden Tabelle zusammengefasst.

### Beispiele 11 bis 15:

Es wird genau wie in Vergleichsbeispiel 1 verfahren jedoch wurden die in Tabelle 2 angegebenen Vernetzer in den dort angegebenen Mengen verwendet.

**Tabelle 2:**

| | Vernetzer | Einsatzmenge | CRC [g/g] | Extract. 16 h [Gew.%] |
|---|---|---|---|---|
| Vergleichs-beispiel 1 | Sartomer® SR 9035 | 4,5 g | 35 | 10% |
| Vergleichs-beispiel 2 | Laromer® TPGDA | 2,1 g | 41 | 19% |
| Beispiel 11 | aus Beispiel 6 | 1,7 g | 36 | 8% |
| Beispiel 12 | aus Beispiel 7 | 3,4 g | 37 | 8% |
| Beispiel 13 | aus Beispiel 8 | 4,4 g | 35 | 6% |
| Beispiel 14 | aus Beispiel 9 | 1,8 g | 37 | 5% |
| Beispiel 15 | aus Beispiel 10 | 3,5 g | 36 | 7% |

Die eingesetzten Mengen sind dabei so berechnet, dass jeweils die gleiche Anzahl an Doppelbindungsäquivalenten bezogen auf Acrylsäuremonomer zur Vernetzung vorliegen. Man erkennt, dass die erfindungsgemäss eingesetzten Vernetzer bei etwas höherer CRC jeweils deutlich niedrigere extrahierbare Polymeranteile aufweisen. Ein solches Verhalten ist vorteilhaft, da die extrahierbaren Polymeranteile die Produkteigenschaften verschlechtern.

## Patentansprüche

1. (Meth)acrylsäureester ungesättigter Aminoalkohole der allgemeinen Formel 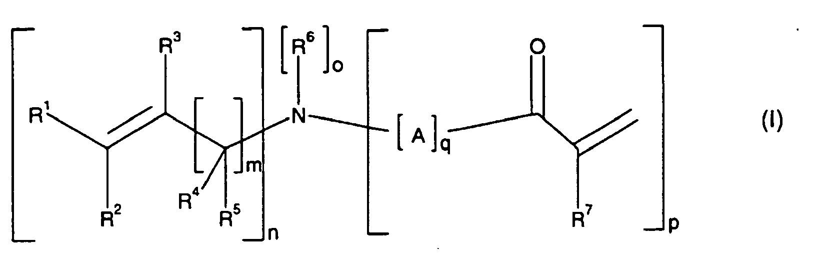 in der
R¹, R², R³, R⁴ und R⁵ unabhängig voneinander Wasserstoff oder C₁- bis C₆-Alkyl, wobei C₃- bis C₆-Alkyl verzweigt oder unverzweigt sein können,
R⁶ C₁- bis C₆-Alkyl, wobei C₃- bis C₆-Alkyl verzweigt oder unverzweigt sein können,
R⁷ Wasserstoff oder Methyl,
m eine ganze Zahl von 0 bis 10,
n 1 oder 2,
o 0 oder 1,
p 1 oder 2 und
q eine ganze Zahl von 2 bis 100
bedeuten,
die Summe von n, o und p gleich 3 ist und
A für gleiche oder verschiedene Reste, ausgewählt aus der Gruppe 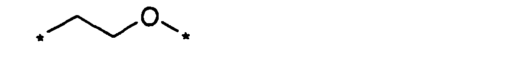 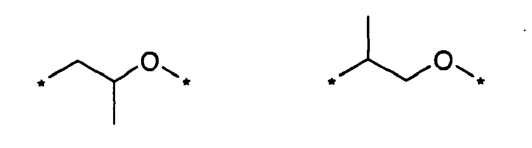 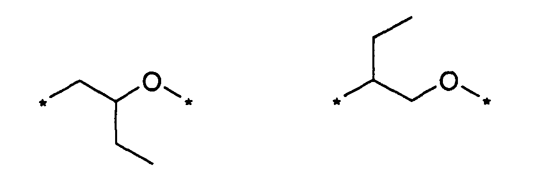 steht und mit ` die Verknüpfungspositionen **gekennzeichnet** sind.

2. (Meth)acrylsäureester ungesättigter Aminoalkohole der allgemeinen Formel gemäß Anspruch 1, in der
R¹, R², R³, R⁴ und R⁵ Wasserstoff,
R⁶ C₁- bis C₃-Alkyl, wobei C₃-Alkyl verzweigt oder unverzweigt sein kann,
R⁷ Wasserstoff oder Methyl,
m 0 oder 1,
n 1 oder 2,
o 0 oder 1,
p 1 oder 2 und
q eine ganze Zahl von 3 bis 40
bedeuten,
die Summe von n, o und p gleich 3 ist und
A für gleiche oder verschiedene Reste, ausgewählt aus der Gruppe 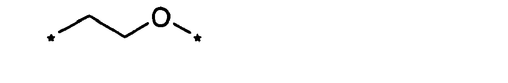 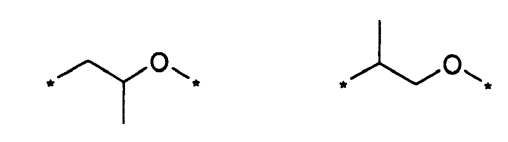 steht und mit * die Verknüpfungspositionen **gekennzeichnet** sind.

3. (Meth)acrylsäureester ungesättigter Aminoalkohole der allgemeinen Formel gemäß Anspruch 1 in der
R¹, R², R³, R⁴ und R⁵ Wasserstoff,
R⁷ Wasserstoff oder Methyl,
m 1,
n 1 oder 2,
o 0,
p 1 oder 2 und
q eine ganze Zahl von 5 bis 20
bedeuten,
die Summe von n, o und p gleich 3 ist und
A 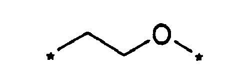 ist und mit ***** die Verknüpfungspositionen **gekennzeichnet** sind.

4. Verfahren zur Herstellung der (Meth)acrylsäureester ungesättigter Aminoalkohole nach Anspruch 1 bis 3, **dadurch gekennzeichnet, dass** man ungesättigte Aminoalkohole mit niederen (Meth)acrylsäureestern in Gegenwart eines Katalysators umestert, während der Reaktion den freigesetzten niederen Alkohol, gegebenenfalls als Azeotrop, und nach beendeter Reaktion den nicht umgesetzten niederen (Meth)acrylsäureester abdestilliert, gegebenenfalls mit Wasser verdünnt und filtriert.

5. Quellbares hydrogelbildendes Polymer, enthaltend einen einpolymerisierten Innenvernetzer der allgemeinen Formel 1 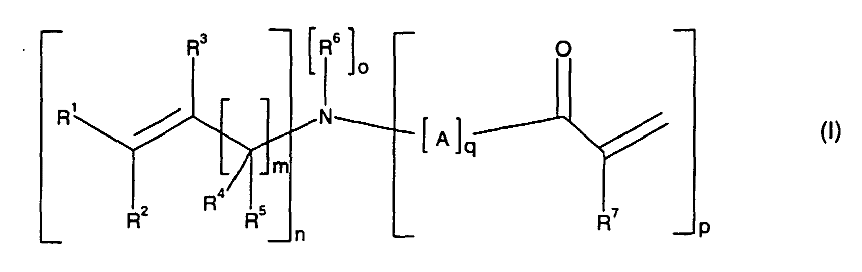 in der
R¹, R², R³, R⁴ und R⁵ unabhängig voneinander Wasserstoff oder C₁- bis C₆-Alkyl, wobei C₃- bis C₆-Alkyl verzweigt oder unverzweigt sein können,
R⁶ C₁- bis C₆-Alkyl, wobei C₃- bis C₆-Alkyl verzweigt oder unverzweigt sein können,
R⁷ Wasserstoff oder Methyl,
m eine ganze Zahl von 0 bis 10,
n 1 oder 2,
o 0 oder 1,
p 1 oder 2 und
q eine ganze Zahl von 1 bis 100
bedeuten,
die Summe von n, o und p gleich 3 ist und
A für gleiche oder verschiedene Reste, ausgewählt aus der Gruppe 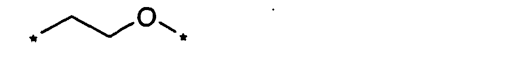 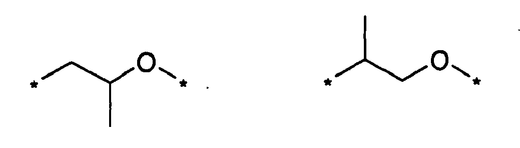 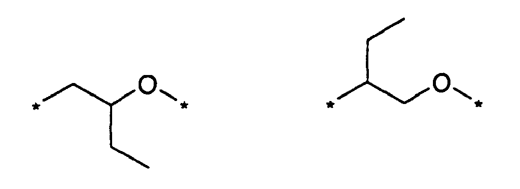 steht.

6. Quellbares hydrogelbildendes Polymer, enthaltend einen einpolymerisierten Innenvernetzer der allgemeinen Formel I nach Anspruch 2.

7. Quellbares hydrogelbildendes Polymer, enthaltend einen einpolymerisierten Innenvernetzer der allgemeinen Formel I nach Anspruch 3.

8. Verfahren zur Herstellung vernetzter quellbarer hydrogelbildender Polymere nach Anspruch 5 bis 7, **dadurch gekennzeichnet, dass** man eine wässrige Mischung, enthaltend ein hydrophiles Monomer, gegebenenfalls mindestens eine weitere monoethylenisch ungesättigte Verbindung, mindestens einen (Meth)acrylsäureester ungsättigter Aminoalkohole, mindestens einen Radikalstarter sowie gegebenenfalls mindestens eine Pfropfgrundlage, polymerisiert und gegebenenfalls das erhaltene Reaktionsgemisch nachvernetzt, trocknet und auf die gewünschte Korngröße bringt.

9. Verwendung von vernetzten quellbaren hydrogelbildenden Polymeren nach Anspruch 5 bis 7 zur Herstellung eines Hygieneartikels.

10. Hygieneartikel, enthaltend ein vernetztes quellbares hydrogelbildendes Polymer nach Anspruch 5 bis 7.

## Claims

1. (Meth)acrylic esters of unsaturated amino alcohols of the general formula I 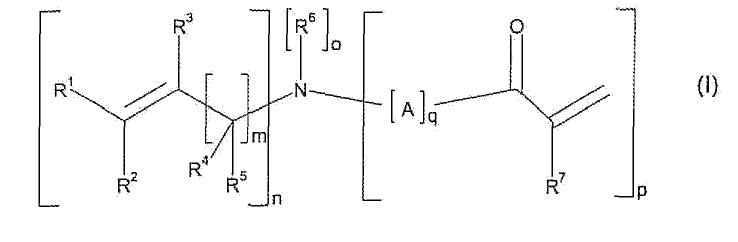 where
R¹, R² , R³, R⁴ and R⁵ are each independently hydrogen or C₁ to C₆ alkyl, of which C₃ to C₆ alkyl may be branched or unbranched,
R⁶ is C₁, to C₆ alkyl, of which C₃ to C₆ alkyl may be branched or unbranched,
R⁷ is hydrogen or methyl,
m is an integer from 0 to 10,
n is 1 or 2,
o is 0 or 1,
p is 1 or 2,
q is an integer from 2 to 100,
the sum total of n, o and p us 3, and
A represents identical or different radicals selected from the group consisting of 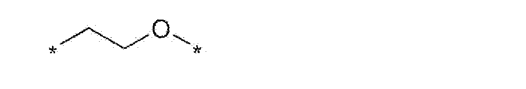 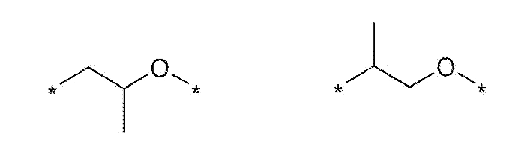 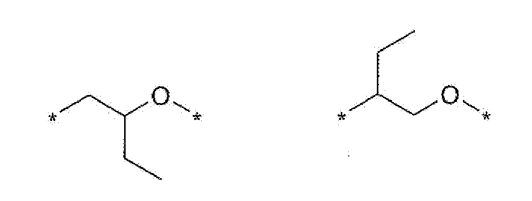 where * identifies the positions of attachment.

2. (Meth)acrylic esters of unsaturated amino alcohols of the general formula I as per claim 1, where
R¹, R², R³, R⁴ and R⁵ are each hydrogen,
R⁶ is C₁ to C₃ alkyl, of which C₃ alkyl may be branched or unbranched,
R⁷ is hydrogen or methyl,
m is 0 or 1,
n is 1 or 2,
o is 0 or 1,
p is 1 or 2,
q is an integer from 3 to 40,
the sum total of n, o and p is 3 and
A represents identical or different radicals selected from the group consisting of 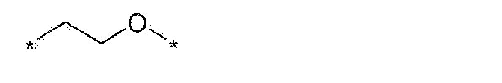 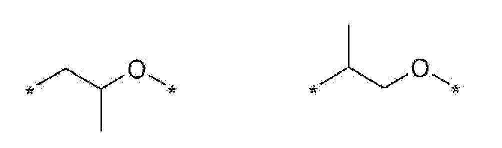 where * identifies the positions of attachment.

3. (Meth)acrylic esters of unsaturated amino alcohols of the general formula I as per claim 1, where
R¹, R², R³, R⁴ and R⁵ are each hydrogen,
R⁷ is hydrogen or methyl,
m is 1,
n is 1 or 2,
o is 0,
p is 1 or 2,
q is an integer from 5 to 20,
the sum of total of n, o and p is 3, and
A is 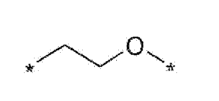 where * identifies the positions of attachment.

4. A process for preparing the (meth)acrylic esters of unsaturated amino alcohols according to claim 1 to 3, which comprises unsaturated amino alcohols being transesterified with lower (meth)acrylic esters in the presence of a catalyst, the released lower alcohol being distilled off daring the reaction, if appropriate as an azeotrope, and the unconverted lower (meth)acrylic ester being distilled off after the reaction has endued , optionally diluted with water and filtered.

5. Swellable hydrogel-forming polymer comprising a copolymerized internal crosslinker of the general formula I 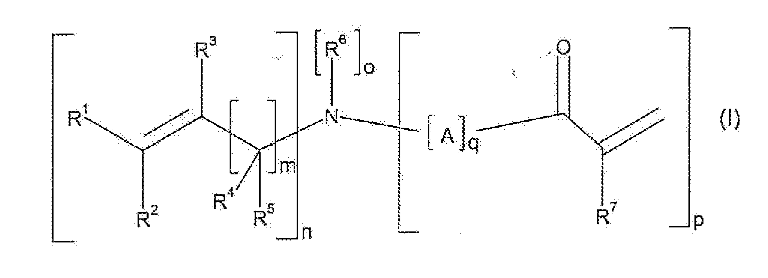 where
R¹, R², R³, R⁴ and R⁵ are each independently hydrogen or C₁ to C₆ alkyl, of which C₃ to C₆ alkyl may be branched or unbranched,
R⁶ is C₁ to C₆ alkyl, of which C₃ to C₆ alkyl may be branched or unbranched,
R⁷ is hydrogen or methyl,
m is an integer from 0 to 10,
n is 1 or 2,
o is 0 or 1,
p is 1 or 2,
q is an integer from 1 to 100,
the sum total of n, o and p is 3, and
A represents identical or different radicals selected from the group consisting of

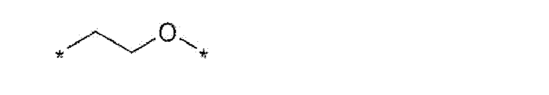

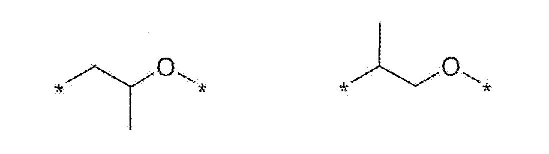

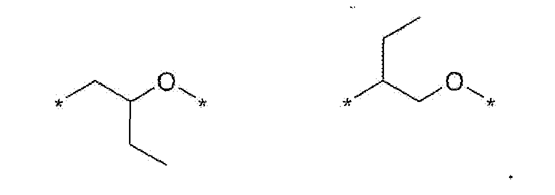

6. Swellable hydrogen-forming polymer comprising a copolymerized internal crosslinker of the general formula I according to claim 2.

7. Swellable hydrogel-forming polymer comprising a copolymerized internal crosslinker of the general formula I according to claim 3.

8. A process for preparing crosslinked swellable hydrogel-forming polymers according to claim 5 to 7, which comprises polymerizing an aqueous mixture comprising a hydrophilic monomer, optionally at least one further monoethylenically unsaturated compound, at least one (meth)acrylic ester of unsaturated amino alcohols, at least one free-radical initiator and optionally also at least one grafting base, and optionally the reaction mixture obtained being postcrosslinked, dried and brought to the desired article size.

9. The ruse of crosslinked swellable hydrogel-forming polymers according to claim 5 to 7 for manufacturing a hygiene article,

10. A hygiene article comprising a crosslinked swellable hydrogel-forming polymer according to claim 5 to 7.

## Revendications

1. Esters de l'acide (méth)acrylique d'aminoalcools insaturées de formule générale I 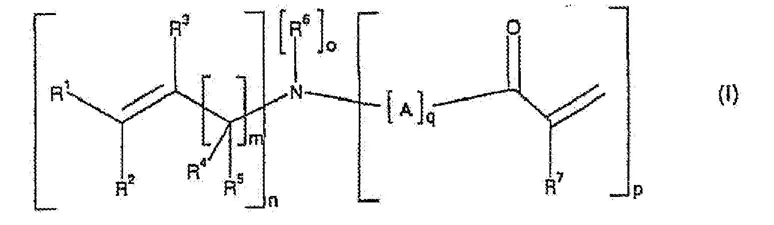 dans laquelle
R¹,R² R³, R⁴ et R⁵ signifient, indépendamment l'un de l'autre, hydrogène ou C₁-C₆-alkyle, où les radicaux G₃-C₆-alkyle peuvent être ramifiés ou non ramifiées,
R⁶ signifie C₁-C₆-alkyle, où les radicaux C₃-C₆-alkyle peuvent être ramifiés ou non ramifiés,
R⁷ signifie hydrogène ou méthyle,
m vaut un nombre entier de 0 à 10,
n vaut 1 ou 2,
o vaut 0 ou 1,
p vaut 1 ou 2 et
q vaut un nombre entier de 2 à 100, la somme de n, o et p vaut 3 et
A représente des radicaux identiques ou différents, choisis dans le groupe 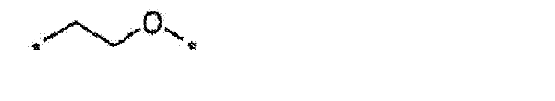 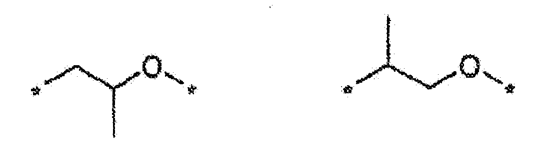 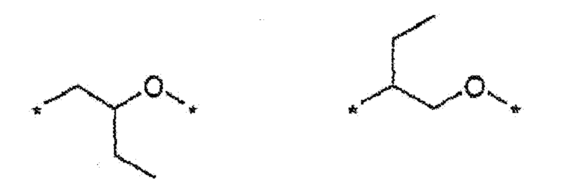
et * caractérise les positions de liaison.

2. Esters de l'acide (méth)acrylique d'aminoalcools insaturés de formule générale I selon la revendication 1, où
R¹,R², R³, R⁴ et R⁵ signifient hydrogène,
R⁶ signifie C₁-C₃-alkyle, où le radical C₃-alkyle peut être ramifié ou non ramifié,
R⁷ signifie hydrogène ou méthyle,
m vaut 0 ou 1,
n vaut 1 ou 2,
o vaut 0 ou 1,
p vaut 1 ou 2 et
q vaut un nombre entier de 3 à 40, la somme de n, o et p vaut 3 et
A représente des radicaux identiques ou différents, choisis dans le groupe 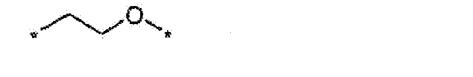 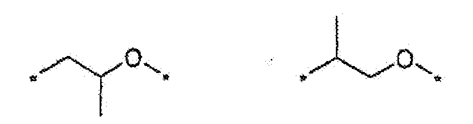
et * caractérise les positions de liaison.

3. Esters de l'acide (méth) acrylique d'aminoalcools insaturés de formule générale I salon la revendication 1, où
R¹, R², R³, R⁴ et R⁵ signifient hydrogène,
R⁷ signifie hydrogène ou méthyle,
m vaut 1,
n vaut 1 ou 2,
o vaut 0,
p vaut 1 ou 2 et
q vaut un nombre entier de 5 à 20, la somme de n, o et p vaut 3 et
A représente 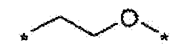 et * caractérise les positions de liaison.

4. Procédé pour la préparation des esters de l'acide (méth)acrylique d'aminoalcools insaturés selon la revendication 1 à 3, **caractérisé en ce qu'**on transestérifie des aminoalcools insaturés avec des esters inférieurs de l'acide (méth)acrylique en présence d'un catalyseur, 1 on élimine par distillation pendant la réaction l'alcool inférieur libéré, le cas échéant sous forme d'azéotrope, et après la réfaction l'ester inférieur de l'acide (méth)acrylique non transformé, le cas échéant on dilue avec de l'eau et on filtre.

5. Polymère gonflable formant un hydrogel, contenant un réticulant interne copolymérisé de formule générale 1 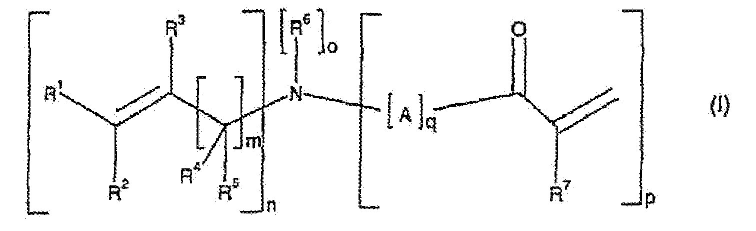 dans laquelle
R¹, R², R³, R⁴ et R⁵ lignifient, indépendamment l'un de l'autre, hydrogène ou C₁-C₆-alkyle, où les radicaux C₃-C₆-alkyle peuvent être ramifiés ou non ramifiés,
R⁶ signifie C₁-C₆-alkyle, où les radicaux C₃-C₆-alkyle peuvent être ramifiés ou non ramifiés,
R⁷ signifie hydrogène ou méthyle,
m vaut un nombre entier de 0 à 10,
n vaut 1 ou 2,
o vaut 0 ou 1,
p vaut 1 ou 2 et
q vaut un nombre entier de 1 à 100,
la somme de n, o et p vaut 3 et
A représente des radicaux identiques ou différents, choisis dans le groupe

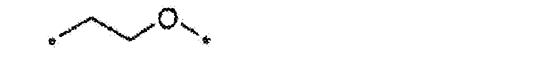

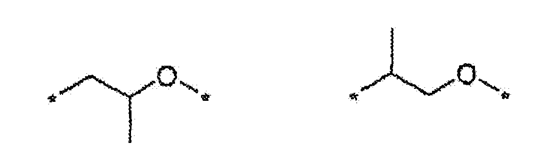

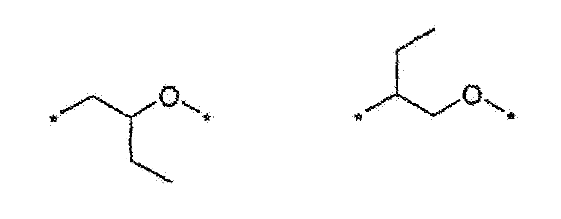

6. Polymère gonflable formant un hydrogel, contenant un réticulant interne copolymérisé de formule générale I selon la revendication 2.

7. Polymère gonflable formant un hydrogel, contenant un réticulant interne copolymérisé de formule générale I selon la revendication 3.

8. Procédé pour la préparation de polymères gonflables réticulés formant un hydrogel selon la revendication 5 à 7, **caractérisé en ce qu'**on polymérise un mélange aqueux, contenant un monomère hydrophile, le cas échéant au moins un autre composé monoéthyléniquement insaturé, au moins un ester de l'acide (méth)acrylique d'aminoalcools insaturés, au moins un initiateur de radicaux ainsi que le cas échéant au moins une base de greffage et le cas échéant on post-réticule le mélange réactionnel obtenu, on le sèche et on l'amène à la granulométrie souhaitée.

9. Utilisation de polymères gonflables réticulés formant un hydrogel selon la revendication 5 à 7 pour la préparation d'un objet destiné à l'hygiène.

10. Objet destiné à l'hygiène, contenant un polymère gonflable réticulé formant un hydrogel selon la revendication 5 à 7.
